# EUROPEAN PATENT APPLICATION

(11) **EP 0 875 246 A1**
(43) Date of publication of application: **04.11.1998**
(21) Application number: 98106276.3
(22) Date of filing: 06.04.1998
(51) Int. Cl.: A61K 31/375, A61K 45/06

(54) **Pharmaceutical preparation of ascorbic acid derivatives for medical treatment of cancer**

(30) Priority: 04.04.1997 JP 102785/97
(71) Applicant: SHOWA DENKO KABUSHIKI KAISHA, Minato-ku, Tokyo (JP)
(72) Inventor: Miwa, Nobuhiko, Shouhara-shi, Hiroshima 727-0013 (JP); Suzuki, Masahiro, Sanbu-gun, Chiba 299-3234 (JP); Tsuzuki, Toshi, c/o Showa Denko K.K., Chiba-shi, Chiba 267-0056 (JP); Tsuchiya, Toshiyuki, c/o Showa Denko K.K., Chiba-shi, Chiba 267-0056 (JP); Itoh, Shinobu, c/o Showa Denko K.K., Tokyo 105-8518 (JP); Ogata, Eiji, c/o Showa Denko K.K., Tokyo 105-8518 (JP)
(74) Representative: Strehl Schübel-Hopf & Partner

(57) **Abstract**

A pharmaceutical preparation comprising at least one L-ascorbic acid derivative and at least one antimalignant tumor agent, wherein said L-ascorbic acid derivative provides, on culturing zooblast for 20 hours in a culture medium containing the L-ascorbic acid derivative in a concentration of 5 µM, an L-ascorbic acid intracellular cumulative amount equal to or greater than that obtained by culturing the zooblast, in a culture medium containing magnesium L-ascorbic acid-2-phosphate in a concentration of 50 µM. Also disclosed is a pharmaceutical preparation, for inhibiting cancer metastasis, which comprises the L-ascorbic acid derivative. Also disclosed is a method of treating cancer which comprises administering to a person in need of such treatment a dosage effective amount of the pharmaceutical preparation.

## Description

### FIELD OF THE INVENTION

The present invention relates to an anticancer agent comprising one or more active ingredients including an L-ascorbic acid derivative which provides stable L-ascorbic acid activity and having improved cell uptake capability, or to a combination or the L-ascorbic acid derivative and a known antimalignant tumor agent. The present invention also relates to a cancer metastasis inhibitor and to a method of treating cancer which comprises administering the cancer metastasis inhibitor alone or in combination with an antimalignant tumor agent.

### BACKGROUND OF THE INVENTION

A large number of cancer metastasis inhibitors have hitherto been proposed (see, e.g., JP-A-54-44011 (the term "JP-A" as used herein means an "unexamined published Japanese patent application"), JP-A-02-3087, JP-A-03-31214, JP-A-03-56417, JP-A-04-77435, JP-A-04-312531, JP-A-06-72871, JP-A-06-107693, JP-A-06-116184, JP-A-06-107548 and JP-A-06-87847). However, the effect of these metastasis inhibitors is not yet satisfactory.

As reported in these patent publications, L-ascorbic acid derivatives have conventionally been known to exhibit anticancer action. In this regard, the following have been proposed. (1) an antitumor agent comprising O-benzylidene-ascorbic acid or a salt thereof (JP-A-60-139619), (2) a medical treating agent composition exhibiting anticancer activity comprising an anticancer platinum complex and L-ascorbic acid, 5,6-O-benzylidene L-ascorbic acid or 5,6-O-benzylidene L-ascorbic acid wherein at least the 1-position of the aldehyde group in the benzylidene moiety is substituted with a heavy hydrogen (JP-A-02-096524), and (3) 5,6-(3-nitro)benzylidene-L-ascorbic acid or a salt thereof (JP-A-05-004985).

However, the L-ascorbic acid derivatives (1), (2) and (3) are easily degraded in vivo by an enzyme or the like and are unstable. Accordingly, a sufficiently high intracellular cumulative amount is not obtained by administering these derivatives. Furthermore, L-ascorbic acid derivatives such as L-ascorbic acid-2-phosphate and L-ascorbic acid-2-glucoside have a low absorption rate into cells and a large dose is required so as to achieve a desired concentration. This gives rise to problems in formulating these derivatives into a pharmaceutical preparation or in administering the same.

In general, L-ascorbic acid derivatives are very prone to oxidation and are unstable due to their strong reducing property. Because they are oxidized during formulation into a pharmaceutical preparation, it is difficult to maintain their quality as a medical preparation. Also, L-ascorbic acid derivatives that are formulated into an injection preparation present a problem in that uptake in a stable state cannot be achieved in vivo by phlebolysis or the like.

Furthermore, even if an L-ascorbic acid derivative is perorally or intravenously administered, its activity readily disappears in vivo and the derivative is swiftly discharged by metabolic action outside the body. Thus, the effect of L-ascorbic acid is not sufficiently exerted.

In order to overcome the problems of these conventional L-ascorbic acid derivative anticancer agents or cancer metastasis inhibitors, the present inventors have proposed a medical preparation comprising a specific L-ascorbic acid derivative as a main component (Japanese Patent Application No. 8-143720). In the invention of Japanese Patent Application No. 8-143720, the use of an L-ascorbic acid-2-phosphate ester as a main component successfully avoids oxidation and stabilizes the L-ascorbic acid. However, the cell absorption rate is low and the intracellular L-ascorbic acid concentration cannot be effectively increased. This result seems to be ascribable to the high hydrophilicity of the L-ascorbic acid-2-phosphate ester.

### SUMMARY OF THE INVENTION

It is therefore an objective of the present invention to provide a cancer metastasis inhibitor comprising an L-ascorbic acid derivative which is stable against oxidation, has excellent absorptivity into zooblast and which can effectively increase the intracellular cumulative amount of L-ascorbic acid. It is furthermore an objective of the present invention to provide a method of treating cancer which comprises administering the cancer metastasis inhibitor.

The above objectives of the present invention are achieved by providing:
(1) A pharmaceutical preparation comprising at least one L-ascorbic acid derivative and at least one antimalignant tumor agent, wherein said L-ascorbic acid derivative provides, on culturing zooblast for 20 hours in a culture medium containing the L-ascorbic acid derivative in a concentration of 5 µM, an L-ascorbic acid intracellular cumulative amount equal to or greater than that obtained by culturing the zooblast in a culture medium containing magnesium L-ascorbic acid-2-phosphate in a concentration of 50 µM.
(2) A pharmaceutical preparation comprising at least one L-ascorbic acid derivative and at least one antimalignant tumor agent, wherein said L-ascorbic acid derivative is represented by the following formula (1) and pharmaceutically acceptable salts thereof and having an ester bond or an ether bond at the 2-position thereof and a hydrophobic group at one or both of the 5- and 6-positions thereof: 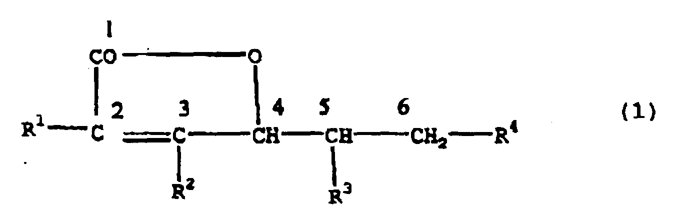 wherein R¹ is a substituent bonded to the 2-position through the ester or ether bond capable of being biotransformed into a hydroxy group; R² represents a hydroxyl group or a substituent capable of being biotransformed into a hydroxyl group; and at least one of R³ and R⁴ is a hydrophobic group and the other is a hydroxyl group or a hydrophobic group.
(3) A method of inhibiting cancer metastasis which comprises administering to a person in need of such treatment a dosage effective amount of at least one L-ascorbic acid derivative which provides, on culturing zooblast for 20 hours in a culture medium containing the L-ascorbic acid derivative in a concentration of 5 µM, an L-ascorbic acid intracellular cumulative amount equal to or greater than that obtained by culturing the zooblast in a culture medium containing magnesium L-ascorbic acid-2-phosphate in a concentration of 50 µM.
(4) A pharmaceutical preparation, for inhibiting cancer metastasis, which comprises at least one L-ascorbic acid derivative represented by the following formula (1) and pharmaceutically acceptable salts thereof, and having an ester bond or an ether bond at the 2-position thereof and a hydrophobic group at one or both of the 5- and 6-positions thereof: 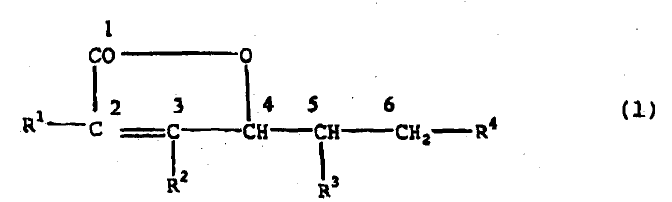 wherein R¹ is a substituent bonded to the 2-position through the ester or ether bond capable of being biotransformed into a hydroxy group; R² represents a hydroxyl group or a substituent capable of being biotransformed into a hydroxyl group; and at least one of R³ and R⁴ in a hydrophobic group and the other is a hydroxyl group or a hydrophobic group.
(5) A method of treating cancer which comprises administering to a person in need of such treatment a dosage effective amount of the pharmaceutical preparation of (2) above.

### DESCRIPTION OF THE PREFERRED EMBODIMENTS

The present inventors have made extensive studies taking into account the above-described problems of the prior art. As a result, the present inventors have found that an L-ascorbic acid derivative which is stabilized, appropriately fat-soluble and easily biotransformed in vivo into L-ascorbic acid, exhibits good cell absorptivity even when present in a low concentration and effectively inhibits cancer metastasis.

This L-ascorbic acid derivative is an L-ascorbic acid derivative such that when, for example, bovine aortic endothelial BAE-2 cells are cultured in a culture medium containing the L-ascorbic acid derivative in a concentration of 5 µM or less for 20 hours, an L-ascorbic acid intracellular cumulative amount is obtained equal to or greater than that obtained when using a culture medium containing 50 µM of magnesium L-ascorbic acid-2-phosphate.

This L-ascorbic acid derivative has a structure, for example, such that an ester bond or an ether bond is present at the 2-position for improving stability, and a hydrophobic group is present at least at the 5- or 6-position for imparting fat solubility and elevating cell membrane permeability.

The substituents of the L-ascorbic acid derivative are not particularly limited, and may be selected depending on the intended purpose or desired effect. However, when a prompt effect is demanded, the substituent at the 2- or 3-position is preferably a substituent capable of being quickly hydrolyzed by an enzyme or the like in blood serum (for example, a phosphoryl group in the case of human beings). On the contrary, when duration is demanded, a substituent which is gradually hydrolyzed is preferred (for example, a glycosyl group in the case of human beings). The substituent at the 5- or 6-position preferably has an appropriate hydrophobicity and is replaced by a hydroxyl group due to the action of an intracellular enzyme so as to attain fast cellular uptake. For example, in the case of human beings, an ester of palmitic acid or lauric acid is preferred.

This L-ascorbic acid derivative of the present invention is a compound represented by formula (1) or a salt thereof:

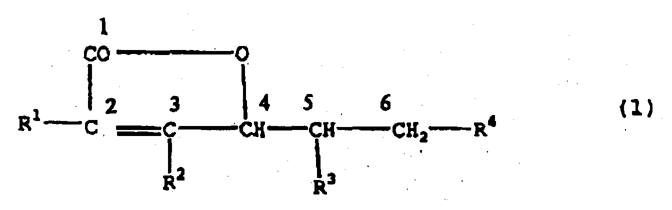

In formula (1), R¹ is a group bonded to the carbon atom at the 2-position of the ascorbic acid through an ester bond or an ether bond and is capable of being biotransformed into a hydroxyl group. Examples of R¹ include a phosphoryloxy group (for example, [-OP(O)(OH)₂]), a pyrophosphoryloxy group (for example, [-OP(O)(OH)OP(O)(OH)₂]), a triphosphoryloxy group (for example, [-O(P(O)(OH)O)₃H] , a polyphosphoryloxy group and a glycosyloxy group. In view of ease and stability in production, a phosphoryloxy group is preferred.

R² is a hydroxyl group or a group capable of being biotransformed into a hydroxyl group, and examples thereof include a hydroxyl group, a phosphoryloxy group, a pyrophosphoryloxy group, a triphosphoryloxy group, a polyphosphoryloxy group, a sulfoxy group, a glycosyloxy group, an alkoxy group, an alkenyloxy group and a phenoxy group. When the compound is derived from L-ascorbic acid in production, no problems generally arise by keeping R² as a hydroxyl group.

At least one of R³ and R⁴ is a substituent containing a hydrophobic group and the other is a hydroxyl group or a substituent containing a hydrophobic group. Examples of the hydrophobic group include an acyloxy group having from 10 to 22 carbon atoms, an alkoxy group, an alkenyloxy group and a phenoxy group. Among these, acyloxy groups of fatty acid esters are preferred, and examples thereof include esters of lauric acid, myristic acid, palmitic acid, stearic acid, oleic acid, linoleic acid, linolenic acid and arachidonic acid.

Specific examples of preferred L-ascorbic acid derivatives include L-ascorbic acid-2-phosphate (including various phosphoric acid esters)-6-higher fatty acid esters (examples of the higher fatty acids include lauric acid, palmitic acid, stearic acid and arachidonic acid) and salts thereof in a physiologically acceptable form.

Examples of the inorganic base for forming the above-described salts include ammonium, an alkali metal (e.g., sodium, potassium), an alkaline earth metal (e.g., calcium, magnesium, strontium, barium) and an aluminum salt, and examples of the organic salt base therefor include trimethylamine, triethylamine, pyridine, picoline, N,N-dibenzylethylenediamine, ethanolamine, diethanolamine, tris-hydroxymethylaminomethane and dicyclohexylamine. From these, one base or a mixture of two or more bases may be selected, however, in view of particularly low cytotoxicity, low cost, high safety and economic advantage, one or a mixture of two or more selected from sodium, potassium, magnesium, calcium and aluminum is preferably used as the salt of the compound represented by formula (1).

Salts of the compound represented by formula (1) are generally easy to dissolve in water and have low cytotoxicity as compared with general antimalignant tumor agents, and a salt thereof having high compatibility with a living body can be freely selected because the pH can be controlled. Accordingly, the salts are more suitable for use in the cancer metastasis inhibitor of the present invention.

Of the L-ascorbic acid-2-phosphate-fatty acid esters represented by formula (1) of the present invention and salts thereof, a cancer metastasis inhibitor comprising one or a mixture of two or more selected from L-ascorbic acid-2-pyrophosphate esters, L-ascorbic acid-2-triphosphate esters, L-ascorbic acid-2-polyphosphate esters and salts thereof, has a low production cost and therefore, is suitable for obtaining a less expensive pharmaceutical preparation for the medical treatment of cancer as compared with other conventional pharmaceutical preparations.

The inventive pharmaceutical preparation for the medical treatment of cancer is generally used as an injection preparation or a peroral preparation and therefore, preferably has a high water solubility. In the L-ascorbic acid-2-phosphate-fatty acid ester represented by formula (1) or a salt thereof, the effective ingredient is preferably a water adduct or a hydrate thereof because its solubility in water is higher. Furthermore, monovalent salts are preferred because the solubility is higher than that of divalent salts. The water content or water of hydration in the water adduct or hydrate is not particularly limited. However, for providing higher solubility when the effective ingredient of the present invention is a solid, the water content is preferably from 1 to 50% (wt/wt) or the hydrate is preferably a monohydrate to an eicosahydrate. In particular, a sodium salt and a potassium salt of such L-ascorbic acid derivatives are suitable as the cancer metastasis inhibitor of the present invention because of their low cytotoxicity and accordingly, high compatibility with a living body.

The cancer metastasis inhibitor of the present invention has low toxicity and can be perorally or parenterally administered to mammals including humans.

The form of the cancer metastasis inhibitor of the present invention is not particularly limited, and can be formulated into various pharmaceutical preparations as a medical product. Examples thereof include a peroral administration form, tablet, powder, solution, suppository, external application form, ointment, eye drop, phleboclysis, powder, granule, tablet, sugar-coated tablet, capsule, pill, suspension, ampule, injection and isotonic solution.

The cancer metastasis inhibitor of the present invention may be formulated as a pharmaceutically acceptable inert single substance or as a mixture with a diluent and/or other pharmacologically active substances, or may be formulated into a dose unit form.

In the present invention, these effective ingredients can be mixed and administered as a one-part preparation according to a known pharmaceutical production process using, if desired, a pharmaceutically acceptable diluent or excipient as described in Japanese Pharmacopeia, 12th ed., Hirokawa Shoten (1991).

Also, the effective ingredients may be separately formulated into respective pharmaceutical preparations using, if desired, a pharmaceutically acceptable diluent or excipient and then administered.

In the case of a solution, the pharmaceutical preparation of the present invention can be formulated by an ordinary method using a solvent such as a water-soluble solvent (e.g., distilled water), a water-soluble preparation (e.g., physiological saline, Ringer's solution) or an oily solvent (e.g., sesame oil, corn oil, olive oil).

In this case, if desired, additives such as a dissolution aid (e.g., sodium salicylate, sodium acetate), a buffer (sodium citrate, glycerin), an isotonic agent (e.g., grape sugar), a stabilizer (e.g., human serum albumin, polyethylene glycol), a preservative (e.g., benzyl alcohol, phenol) and an analgesic (e.g., benzalkonium chloride, procaine acetate) may be used.

Furthermore, the pharmaceutical preparation may also be formulated, if desired, by mixing thereto or using a pharmacologically or pharmaceutically acceptable additive (e.g., a diluent, excipient, binder, disintegrator, colorant, stabilizer, extender, wetting agent, surface active agent, lubricant, dispersant, buffer, flavor, fragrance, perfume, preservative, dissolution aid, solvent, coating, sugar-coating).

The diluent for use in the preparation of the cancer metastasis inhibitor of the present invention is a pharmaceutically acceptable substance. The diluent is a material other than the compound of the present invention, and various materials may be used therefor, such as a solid, semisolid liquid or absorbable capsule, a dextrin subsumptive substance and a microcapsule using a phospholipid.

The cancer metastasis inhibitor of the present invention may be produced by a known method. For example, active components may be mixed with a diluent, for example, into a granule and a composition thereof is formed, for example, into a tablet.

The preparation for parenteral administration must be sterile and if desired, isotonic with the blood. The parenteral administration includes administration by means of injection (including, for example, muscular drip phleboclysis) and rectal administration (suppository).

The L-ascorbic acid derivative represented by formula (1) itself is useful as a cancer metastasis inhibitor and in a pharmaceutical preparation or composition thereof, the L-ascorbic acid derivative as an active component is generally contained in an amount of from 0.01 to 100% by weight.

Examples of the composition for peroral administration further include a tablet, pill, granule, powder, capsule, syrup, emulsion, suspension and nebula. These compositions can be produced by a known method using a carrier or excipient such as lactose, starch, sucrose or magnesium stearate.

For parenteral administration, for example, an injection, a suppository, a plaster, an ophthalmic solution or a preparation for external application may be used. The injection is usually filled in an appropriate ampule. The suppository includes an endorectal suppository and a vaginal suppository. The preparation for external application includes an ointment, a nasal administration agent and a peroral administration agent.

For formulating a preparation for external application, the composition of the present invention can be formed into a solid, semisolid or liquid solvent thereof according to a known method. For example, in the case of a solid, the composition of the present invention is processed into a powder composition as such or after adding and mixing thereto an excipient (e.g., glycol, mannitol, starch, microcrystal, cellulose) or a thickener (e.g., natural gum, cellulose derivative, acryl polymer).

Similar to the case of an injection, the liquid can be formed as an oily or aqueous suspension. In the case of a semisolid, an aqueous or oily gel or an ointment is preferred.

In any case, a pH adjusting agent (e.g., carbonic acid, phosphoric acid, hydrochloric acid, sodium hydroxide) and an antiseptic (e.g., para-hydroxybenzoic acid esers, chlorobutanol, benzalkonium chloride) may be added. For obtaining a suppository, the composition of the present invention may be formed into an oily or aqueous solid, semisolid or liquid suppository according to a known method.

The dose of the L-ascorbic acid derivative represented by formula (1) of the present invention or a salt thereof varies depending on the symptom, age, sex, body weight, preparation form or administration form. However, in the case of peroral administration, suppository administration or external application, the dose is usually from 0.001 to 8,500 mg, preferably from 1 to 100 mg, for an adult per 1 kg of the body weight per day, and in the case of phleboclysis or drip, it in from 0.025 to 200 mg, preferably from 0.25 to 100 mg, for an adult per 1 kg of the body weight per day. The preparation may be administered at once or in several parts.

The cancer metastasis of the present invention is useful for the treatment of mammals (e.g., mouse, rat, swine, raccoon dog, fox, cat, rabbit, dog, cattle, horse, goat, monkey, human) suffering from a tumor, and exerts outstanding effects on the treatment of cancer, prolongation of life and inhibition of cancer metastasis in the cancer-carrying animal.

The pharmaceutical preparation for the medical treatment of cancer of the present invention is effective as a cancer metastasis inhibitor and examples of the indication include various malignant tumors and benign tumors such as malignant melanoma, malignant lymphoma, digestive apparatus cancer, pulmonary cancer, esophageal cancer, stomach cancer, large intestine cancer, rectum cancer, colon cancer, ureter tumor, cholecyst cancer, bile duct cancer, biliary cancer, breast cancer, liver cancer, pancreas cancer, testicle cancer, upper jaw cancer, tongue cancer, lip cancer, stomatic cancer, throat cancer, larynx cancer, ovary cancer, uterus cancer, prostate cancer, thyroid gland cancer, brain tumor, Kaposi's sarcoma, hemangioma, leukemia, true hypervolemia, neuroblastoma, retinoblastoma, myeloma, urinary bladder sarcoma, sarcoma, osteosarcoma, myosarcoma, skin cancer, basal cell cancer, skin appendage's cancer, skin metastasis cancer and skin melanoma.

The cancer metastasis inhibitor of the present invention is particularly effective in treating malignant malanoma, digestive apparatus cancer, pulmonary cancer, esophageal cancer, stomach cancer, large intestine cancer, rectum cancer, colon cancer, breast cancer, upper jaw cancer, tongue cancer, lip cancer, stomatic cancer, throat cancer, uterus cancer, Kaposi*'*s sarcoma, hemangioma, retinoblastoma, myosacroma, skin cancer, basal cell cancer, skin appendage*'*s cancer, skin metastasis cancer and skin melanoma, of which the focus part is relatively easily directly contacted with the cancer metastasis inhibitor.

The L-ascorbic acid derivative or a salt thereof as an effective ingredient of the cancer metastasis inhibitor of the present invention has been confirmed to exhibit a synergistic effect on the treatment of cancer and on cancer metastasis inhibitory action when used in combination with specific known antimalignant tumor agents.

Examples of known antimalignant tumor agents which exhibit such a synergistic effect include Nitromin (R), cyclophosphamide, merphalan, TIOTEBA, CARBOCON, Protecton (R), bulsfan, nimustine hydrochloride, MITOPURNITOL, ifosfamide, meercaptopurine, thioinosine, cytarabine, decarbazine, fluorouracil, tegaful, ANCITABINE hydrochloride, methotrexate, carmofur, UFT (R), enocitabine, vinblastine sulfate, vincristine sulfate, vindesine sulfate, actinomycin (D), mitomycin C, chromomycin A3, pleomycin hydrochloride, PLEOMYCIN sulfate, daunorubicin hydrochloride, doxorubicin hydrochloride, neocarzinostatin, vebromycin sulfate, aclarubicin hydrochloride, mebitiostan, epitiostanol, tamoxifen citrate, HONPAN, VICIPANIL (R), krestin, lentinan, L-asparginase, aceglatone, procarbazine hydrochloride, floxuridine, MDS KOWA 3000 (R), Estracyt (R), CIZOFERAN, ADRIAMYCIN, mitomycin, cisplatin, CARBOPLATIN, vindesine, vincristine, cyclophosphamido, IFOMAMIDE, pleomycin, pepleomycin, etoposide and Furtulon. These antimalignant tumor agents can used individually or in a combination of two or more thereof. These agents are administered in a respective dose proposed by the commercial suppliers, as described in the publication, Iyakuhin Yoran, 5th Edition, Yakugyo Jiho-Sha (1992).

Also, the cancer metastasis inhibitory affect can also be increased by using the effective ingredient of the present invention in combination with recently reported antimalignant tumor agents such as angiostatic steroids in the co-presence of protamine or heparin, polysaccarides such as peptidoglycan complex, laminin peptides such as Cys-Asp-Pro-Gly-Tyr-Ile-Gly-Ser-Arg-NH₂ (CDPGYGSR-NH₂), peptides containing an Arg-Gly-Asp-(R GD) sequence, Platelet Factor-4, and anticancer agents including interferon obtained naturally or by a genetic engineering technique.

The L-ascorbic acid derivative or a salt thereof as the effective ingredient of the cancer metastasis inhibitor of the present invention inhibits oxidation degradation of the ascorbic acid and stabilizes the same because of the presence of an ester bond or ether bond (for example, phosphoric ester) introduced at the 2-position of the L-ascorbic acid. Accordingly, aging degradation hardly occurs during the production or storage of the pharmaceutical preparation.

The ascorbic acid derivative in general after uptake in vivo is readily degraded particularly by a radical contained in food or the like present in the digestive tract and cannot reach the target cell. However, the L-ascorbic acid derivative of the present invention is stable in vivo and fat-soluble. Accordingly, it is quickly absorbed by a nutrition absorbing cell or the like.

According to the studies conducted by the present inventors, the L-ascorbic acid derivative of the present invention exhibits very high cell absorptivity as compared with an L-ascorbic acid-2-phosphate or a salt thereof. Furthermore, the present inventors confirmed that a higher intracellular concentration of L-ascorbic acid can be achieved with a 1/10 or less dose as compared with L-ascorbic acid-2-phosphate or a salt thereof.

Thus, it is considered that the L-ascorbic acid derivative represented by formula (1) of the present invention can exert its cancer metastasis inhibitory action with a very small dose unrealizable by L-ascorbic acid-2-phosphate or a salt thereof.

The cancer cell generally has a migratory property and accordingly, can move from the focus into the blood. As a result, the cancer cells migrate, metastasize to a wide range of organs, increase and considerably abridge life.

The present inventors also confirmed that the cancer metastasis inhibitor of the present invention exerts an antitumor action of inhibiting migration of cancer cells from the focus to the angioendothelium to thereby inhibit cancer metastasis; that cancer cells are greatly reduced in wetting ratio to the angioendothelium on administering the L-ascorbic acid derivative of the present invention; and that this action is not provided by ordinary L-ascorbic acid but is specific to the L-ascorbic acid derivative of the present invention.

The L-ascorbic acid derivative as the effective ingredient of the cancer metastasis inhibitor of the present invention is protected from oxidative degradation and stabilized due to the substituent bonded to the 2-position of the L-ascorbic acid through an ester or ether bond.

Furthermore, the L-ascorbic acid derivative of the present invention has a hydrophobic group introduced at least at the 5- or 6-position, whereby the lipophilicity is increased, cell absorptivity is improved and the intracellular L-ascorbic acid concentration is efficiently elevated. As a result, the L-ascorbic acid can maximally exercise its antitumor action. It is considered that the antitumor action of L-ascorbic acid particularly prevents wetting of the tumor cell to the angioendothelium and in turn, inhibits the metastasis of cancer, thereby attaining the anticancer effect.

### EXAMPLES

The present invention is described by reference to the following Examples and Comparative Examples, however, the present invention should not be construed as being limited thereto.

The L-ascorbic acid-2-phosphate-6-carboxylic acid ester or a salt thereof used in the Examples was prepared by the following method.

A carboxylic acid or an ester or salt thereof was reacted with a commercially available L-ascorbic acid-2-phosphate or a salt thereof in the presence of a catalyst such as a dehydration catalyst to obtain an L-ascorbic acid-2-phosphate-6-carboxylic acid ester or a salt thereof. The catalyst used in the reaction was concentrated sulfuric acid, and the reaction temperature was from 5 to 70°C.

As a specific example, the synthesis method of L-ascorbic acid-2-phosphate-6-palmitate is described in greater detail below.

10 mmol of magnesium L-ascorbic acid-2-phosphate was dissolved in 60 ml of concentrated sulfuric acid at room temperature, and 15 mmol of palmitic acid was added to the resulting solution. The mixed solution was thoroughly stirred and left standing at room temperature for 24 hours. Then, the reaction mixture was poured into 300 ml of ice water and the precipitate was extracted twice with 200 ml of diethyl ether.

The extract solution was washed with 300 ml of 2N hydrochloric acid containing 30% isopropanol and diethyl ether was removed by distillating under reduced pressure. The deposit was washed twice with n-hexane and dried under reduced pressure to obtain 3.2 g of the objective L-ascorbic acid-2-phosphate-6-palmitate.

The 6-O-pivaloyl-L-ascorbic acid-3-phosphate was synthesized according to the method described in JP-B-03-055470 (the term "JP-B" as used herein means an "examined Japanese patent publication"). The L-ascorbic acid-5,6-O-benzylidene derivatives were synthesized according to the methods described in JP-A-08-269074, JP-A-01-313476 and JP-A-05-004985. The carboxylic acid ester of 2-O-D-glucopyranosil-L-ascorbic acid was extracted from UV White (trade name, produced by Shiseido Co., Ltd.). Other reagents used were commercially available products.

### Example 1

About 6×10⁵ cells of a normal human adult mamma epidermis keratinocyte (available from Kurashiki Boseki KK) were sowed on a 60 mm plate and cultured in a culturing medium (manufactured by Krashiki Boseki KK) for the increase of serum-free epidermis keratinocyte for 2 hours. After displacing with the same culturing medium having added thereto from 2 to 50 µM of ascorbic acid or a derivative thereof, the cells were further cultured for 20 hours. The cells were collected by trypsin treatment and the number of cells was counted using a Coulter Counter Model DN (trade name, manufactured by Coulter Electronics). The cells thus collected were washed with Hank*'*s equilibrium salt solution, pulverized in an ultrasonic homgenizer, filtered through a Mol-cut II (UFP1LCC) (trade name, produced by Nippon Milipore KK), analyzed by HPLC using Shodex ODSpak F4llA column (trade name, produced by Showa Denko KK), and the amount of ascorbic acid and derivatives thereof were measured to determine the intracellular cumulative amount.

As a control, culturing was performed in the same manner as above except for not adding ascorbic acid or a derivative thereof, and the amount of intracellular ascorbic acid was quantitatively determined.

Table 1 shows the difference in the intracellular ascorbic acid cumulative amount between the case where an ascorbic acid or a derivative thereof was added and the case where an ascorbic acid or a derivative thereof was not added.

**Table 1**

| Ascorbic Acid (Derivative) | Addition Amount | Intracellular Cumulative Amount Pmole/cell |
|---|---|---|
| Sodium L-ascorbic acid 2-phosphate-6-palmitate | 2 µM | 0.0040 |
| Magnesium L-ascorbic acid 2-phosphate-6-laurate | 2 µM | 0.0033 |
| L-Ascorbic acid | 2 µM | <0.0002 |
| Magnesium L-ascorbic acid-2-phosphate | 2 µM | <0.0002 |
| Magnesium L-ascorbic acid 2-phosphate | 10 µM | 0.0003 |
| Magnesium L-ascorbic acid 2-phosphate | 50 µM | 0.0022 |
| Not added | | 0.0000 |

### Example 2

About 6×10⁵ bovine aortic endothelial BAE-2 cells (available from Hiroshima Prefecture University) were sowed on a 60 mm⌀ plate and cultured in a minimum essential culturing medium containing 20% dialyzed bovine fetus serum (available from Moregate) for 2 hours. After displacing with the same culturing medium containing 5 µM (in the case of magnesium ascorbic acid-2-phosphate, 50 µM) of ascorbic acid or a derivative thereof, the cells were further cultured for 20 hours.

Thereafter, the cells were treated in the same manner as in Example 1, and the analysis results of the ascorbic acid and ascorbic acid derivative intracellular contents are shown in Table 2.

**Table 2**

| Ascorbic Acid (Derivative) | Intracellular Cumulative Amount Pmole/cell |
|---|---|
| Sodium L-ascorbic acid 2-phosphate-6-palmitate | 0.0068 |
| Magnesium L-ascorbic acid 2-phosphate-6-laurate | 0.0070 |
| L-Ascorbic acid 2-phosphate-6-palmitate | 0.0072 |
| L-Ascorbic acid 2-phosphate-6-laurate | 0.0042 |
| L-Ascorbic acid 2-phosphate-6-stearate | 0.0034 |
| L-Ascorbic acid 2-phosphate-6-oleate | 0.0055 |
| L-Ascorbic acid 2-phosphate-6-arachidonate | 0.0068 |
| L-Ascorbic acid 2-phosphate-6-benzoate | 0.0025 |
| 6-O-Pivaloyl-L-ascorbic acid-3-phosphate | 0.0009 |
| L-Ascorbic acid | <0.0003 |
| Sodium L-ascorbic acid-2-sulfate | <0.0003 |
| 2-O-D-Glucopyranosil-L-ascorbic acid | <0.0003 |
| L-Ascorbic acid-6-palmitate | 0.0015 |
| L-Ascorbic acid-2,6-dipalmitate | <0.0003 |
| L-Ascorbic acid-5,6-O-benzylidene | 0.0012 |
| 5,6-O-Benzylidene-3-O-[5,7,7-trimethyl-2-(1,3,3-trimethylbutyl)]ocatanoylascorbic acid | 0.0008 |
| Sodium 5,6-(3-nitro)benzylidene-L-ascorbate | 0.0007 |
| Magnesium L-ascorbic acid-2-phosphate | 0.0019 |
| Not added | <0.0003 |

### Example 3

1×100,000 spontaneous breast cancer rat cells (SST-2) were subcutaneously implanted into the back of a rat (SHR rat) (5 rats per group) suffering from spontaneous hypertension. After 35 days, the rats were sacrificed, and the lung weight and the number of colonies formed were measured to examine the degree of metastasis of SST-2 to the lung. The compounds shown in Tables 3 and 4 each was administered perorally or through subcutaneous injection in a dose of 1 mg/rat over the period from the day of implantation to 34 days after implantation. The number of colonies formed was compared with that in the control group not subjected to any treatment, to examine metastasis to the lung. The results are shown in Table 3.

As seen from the results of Examples 1 and 2, the ascorbic acid derivatives (concentration in culturing medium: 5 µM or less) capable of attaining an intercellular ascorbic acid cumulative amount equal to or greater than that obtained in the case of using magnesium L-ascorbic acid-2-phosphate (concentration in culturing medium: 50 µM), provide a strong cancer metastasis inhibitory action. These derivatives are all within the scope formula (1).

**Table 3**

| Compound Administered | Number of Metastasis Colonies (average) | |
|---|---|---|
| | Peroral Administration | Subcutaneous Injection |
| Not added | 88.2 | 88.22 |
| Sodium L-ascorbic acid 2-phosphate-6-palmitate | 30.6 | 1.6 |
| L-Ascorbic acid 2-phosphate-6-palmitate | 34.2 | 2.4 |
| L-Ascorbic acid 2-phosphate-6-laurate | 48.0 | 26.8 |
| L-Ascorbic acid 2-phosphate-6-stearate | 14.2 | 8.2 |
| L-Ascorbic acid 2-phosphate-6-oleate | 29.4 | 2.8 |
| L-Ascorbic acid 2-phosphate-6-arachidonate | 31.4 | 1.2 |
| L-Ascorbic acid 2-phosphate-6-benzoate | 80.0 | 64.4 |
| 6-O-Pivaloyl-L-ascorbic acid-3-phosphate | 89.6 | 86.2 |
| L-Ascorbic acid | 90.8 | 88.8 |
| Sodium L-ascorbic acid-2-sulfate | 86.2 | 85.4 |
| 2-O-D-Glucopyranosil-L-ascorbic acid | 87.2 | 90.6 |
| L-Ascorbic acid-6-palmitate | 85.4 | 83.0 |
| L-Ascorbic acid-2,6-dipalmitate | 89.8 | 86.6 |
| L-Ascorbic acid-5,6-O-benzylidene | 85.8 | 84.2 |
| 5,6-O-benzylidene-3-O-[5,7,7-trimethyl-2-(1,3,3-trimethylbutyl)]octanoylascorbic acid | 87.4 | 89.6 |
| Sodium 5,6-(3-nitro)benzylidene-L-ascorbate | 88.6 | 84.2 |
| Magnesium L-ascorbic acid-2-phosphate | 89.4 | 86.8 |

### Example 4

1×100,000 spontaneous breast cancer rat cells (SST-2) were subcutaneously implanted into the back of a rat (SHR rat) (5 rats per group) suffering from spontaneous hypertension. After 35 days, the rats were sacrificed, and the number of colonies formed were measured to examine the degree of metastasis or SST-2 to the lung.

The ascorbic acid derivatives used were L-ascorbic acid-2-phosphate-6-palmitate, L-ascorbic acid-6-benzoate (reference compound) and magnesium L-ascorbic acid-2-phosphate.

Each of the groups treated with an ascorbic acid derivative was subjected to a test of administering the ascorbic acid derivative alone and to a test of administering the ascorbic acid derivative in combination with a known anticancer agent as shown in Table 4. Furthermore, tests of administering the anticancer agents alone were conducted. Over the period of from 7 days prior to implantation to 34 days after the implantation, the ascorbic acid derivative was injected through a vein in the tail region at a dose of 1 mg/1 day/rat. Each of the known anticancer agents was perorally administered in a minimum dose by reference to the concentration for a single use case described in the publication, Iyakuhin Yoran (Medical Product Handbook), 4th Edition, pp. 1474-1509, Yakugyo Jiho-Sha (1989), and the number of colonies formed after metastasis was compared. The mark "+" indicates that the cancer metastasis inhibitory effect was observed as compared with both of the tests administering the corresponding ascorbic acid derivative alone and the anticancer agent alone, "-" indicates that the effect was not observed as compared with one or both of these tests, and "±" indicates that no judgement could be made.

From these results, the present inventors confirmed that the L-ascorbic acid derivatives shown to have a cancer metastasis inhibitory action in Example 3 exhibited a yet higher cancer metastasis inhibitory effect when used in combination with a known anticancer agent, as compared with administering the L-ascorbic acid derivative alone or the known anticancer agent alone.

**Table 4-1**

| Known Antimalignant Tumor Agent used in combination with the Compound of the Invention | Ascorbic Acid Derivative | | |
|---|---|---|---|
| | L-Ascorbic Acid-2-Phosphate-6-Palmitate | L-Ascorbic Acid-2-Phosphate-6-Benzoate | L-Ascorbic Acid-6-Palmitate |
| Nitromin (R) | + | + | - |
| cyclophosphamido | + | + | - |
| merphalan | + | + | - |
| TIOTEBA | + | + | - |
| CARBOCON | + | + | - |
| Protecton (R) | + | + | - |
| bulsfan | + | ± | - |
| nimustine hydrochloride | + | + | - |
| MITOPURNITOL | + | + | - |
| ifosfamide | + | + | - |
| meercaptopurine | + | + | - |
| thioinosine | + | + | - |
| cytarabine | + | + | - |
| decarbazine | + | + | - |
| fluorouracil | + | + | - |
| tegaful | + | + | - |
| ANCITABINE hydrochloride | + | + | - |
| methotrexate | + | + | - |
| carmofur | + | + | - |
| UFT (R) | + | + | - |
| enocitabine | + | ± | - |

**Table 4-2**

| Known Antimalignant Tumor Agent used in combination with the Compound of the Invention | Ascorbic Acid Derivative | | |
|---|---|---|---|
| | L-Ascorbic Acid-2-Phosphate-6-Palmitate | L-Ascorbic Acid-2-Phosphate-6-Benzoate | L-Ascorbic Acid-6-Palmitate |
| vinblastine sulfate | + | + | - |
| vincristine sulfate | + | + | - |
| vindesine sulfate | + | + | - |
| actinomycyin (D) | + | + | - |
| mitomycin C | + | + | - |
| chromomycin A3 | + | + | - |
| pleomycin hydrochloride | + | + | - |
| PLEOMYCIN sulfate | + | + | - |
| daunorubicin hydrochloride | + | + | - |
| doxorubicin hydrochloride | + | + | - |
| neocarzinostatin | + | + | - |
| vebromycin sulfate | + | + | - |
| aclarubicin hydrochloride | + | + | - |
| mebitiostan, | + | + | - |
| epitiostanol | + | + | - |
| tamoxifen citrate | + | + | - |
| HONPAN | + | + | - |
| VICIPANIL (R) | + | + | - |
| krestin, | + | + | - |
| lentinan | + | + | - |

**Table 4-3**

| Known Antimalignant Tumor Agent used in combination with the Compound of the Invention | Ascorbic Acid Derivative | | |
|---|---|---|---|
| | L-Ascorbic Acid-2-Phosphate-6-Palmitate | L-Ascorbic Acid-2-Phosphate-6-Benzoate | L-Ascorbic Acid-6-Palmitate |
| L-asparginase | + | + | - |
| aceglatone | + | + | - |
| procarbazine | + | + | - |
| hydrochloride floxuridine | + | + | - |
| MDS KOWA 3000 (R) | + | + | - |
| cisplatin | + | + | - |
| Estracyt (R) | + | + | - |
| CIZOFERAN | + | + | - |
| protamine | + | + | - |
| heparin-containing angiostatic steroids | + | + | - |
| peptidoglycan | + | + | - |
| CDPGYGSR-NH₂ | + | + | - |
| anticancer polypeptide | + | ± | - |
| Platelet Factor-4 | + | + | - |

The present invention relates to a cancer metastasis inhibitor and treatment method comprising an L-ascorbic acid derivative having an ester bond or ether bond at the 2-position for preventing hydrolysis in vivo, and a group having an appropriate hydrophobicity at least at the 5-position or 6-position. The L-ascorbic acid derivative has excellent stability in vivo and improved cell uptake capability as compared with other L-ascorbic acid derivatives.

The pharmaceutical preparation for the medical treatment of cancer of the present invention is inexpensive, free of cytotoxicity, and economically advantageous. Also, by using the L-ascorbic acid derivative of the present invention in combination with a wide range of antimalignant tumor agents, the present inventors provide a synergistic effect with respect to suppressing metastasis. Therefore, the inventive pharmaceutical preparation and method of treating cancer is useful for treating malignant tumors and the like, and exhibits strong cancer metastasis inhibiting properties.

While the invention has been described in detail and with reference to specific embodiments thereof, it will be apparent to one skilled in the art that various changes and modifications can be made therein without departing from the spirit and scope thereof.

## Claims

1. A pharmaceutical preparation comprising at least one L-ascorbic acid derivative and at least one antimalignant tumor agent, wherein said L-ascorbic acid derivative provides, on culturing zooblast for 20 hours in a culture medium containing the L-ascorbic acid derivative in a concentration of 5 µM, an L-ascorbic acid intracellular cumulative amount equal to or greater than that obtained by culturing the zooblast in a culture medium containing magnesium L-ascorbic acid-2-phosphate in a concentration of 50 µM.

2. The pharmaceutical preparation according to claim 1 for the medical treatment of cancer.

3. A pharmaceutical preparation comprising at least one L-ascorbic acid derivative and at least one antimalignant tumor agent, wherein said L-ascorbic acid derivative is represented by the following formula (1) and pharmaceutically acceptable salts thereof: 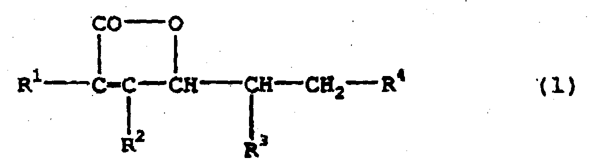 wherein R¹ is a substituent bonded to the 2-position capable of being biotransformed into a hydroxy group; R² represents a hydroxyl group or a substituent capable of being biotransformed into a hydroxyl group; and at least one of R³ and R⁴ is a hydrophobic group and the other is a hydroxyl group or a hydrophobic group.

4. The pharmaceutical preparation according to claim 3, wherein in formula (1), R¹ is one of a phosphoryloxy group, a pyrophosphoryloxy group, a triphosphoryloxy group, a polyphosphoryloxy group, a sulfoxy group and a glycosyloxy group, R² is one of a hydroxyl group, a phosphoryloxy group, a polyphosphoryloxy group, a sulfoxy group, a glycosyloxy group, an alkoxy group, an alkenyloxy group and a phenoxy group, and at least one of R³ and R⁴ is an acyloxy group, an alkoxy group, an alkenyloxy group or a phenoxy group.

5. The pharmaceutical preparation according to claim 3, wherein in formula (1), R¹ is a phosphoryloxy group, and R⁴ is an acyloxy group having from 10 to 22 carbon atoms.

6. The pharmaceutical preparation according to claim 3, wherein the hydrophobic group is selected from the group consisting of acyloxy groups of lauric acid, myristic acid, palmitic acid, stearic acid, oleic acid, linoleic acid, linolenic acid and arachidonic acid.

7. The pharmaceutical preparation according to claim 3, wherein the L-ascorbic acid derivative comprises on L-ascorbic acid-2-phosphate-6-higher fatty acid ester and pharmaceutically acceptable salts thereof, and said higher fatty acid ester is selected from the group consisting of fatty acid esters of lauric acid, palmitic acid, stearic acid and arachidonic acid.

8. The pharmaceutical preparation according to claim 3, wherein the L-ascorbic acid derivative is selected from the group consisting of L-ascorbic acid-2-pyrophosphate esters and pharmaceutically acceptable salts thereof, L-ascorbic acid-2-triphosphate esters and pharmaceutically acceptable salts thereof and L-ascorbic acid-2-polyphosphate esters and pharmaceutically acceptable salts thereof.

9. The pharmaceutical preparation according to claim 3, wherein the L-ascorbic acid derivative is selected from the group consisting or sodium L-ascorbic acid-2-phosphate-6-palmitate, L-ascorbic acid-2-phosphate-6-palmitate, L-ascorbic acid-2-phosphate-6-stearate, L-ascorbic acid-2-phosphate-6-oleate and L-ascorbic acid-2-phosphate-6-arachidonate.

10. The pharmaceutical preparation according to claim 3, wherein said antimalignant tumor agent is selected from the group consisting of Nitromin (R), cyclophosphamide, merphalan, TIOTEBA, CARBOCON, Protecton (R), bulsfan, nimustine hydrochloride, MITOPURNITOL, ifosfamide, meercaptopurine, thioinosine, cytarabine, decarbazine, fluorouracil, tegaful, ANCITABINE hydrochloride, methotrexate, carmofur, UFT (R), enocitabine, vinblastine sulfate, vincristine sulfate, vindesine sulfate, actinomycin (D), mitomycin C, chromomycin A3, pleomycin hydrochloride, PLEOMYCIN sulfate, daunorubicin hydrochloride, doxorubicin hydrochloride, neocarzinostatin, vebromycin sulfate, aclarubicin hydrochloride, mebitiostan, epitiostanol, tamoxifen citrate, HONPAN, VICIPANIL (R), krestin, lentinan, L-asparginase, aceglatone, procarbazine hydrochloride, floxuridine, MDS KOWA 3000 (R), Estracyt (R), CIZOFERAN, ADRIAMYCIN, mitomycin, cisplatin, CARBOPLATIN, vindesine, vincristine, cyclophosphamido, IFOMAMIDE, pleomycin, pepleomycin, etoposide and Furtulon.

11. A method of inhibiting cancer metastasis which comprises administering to a person in need of such treatment a dosage effective amount of at least one L-ascorbic acid derivative which provides, on culturing zooblast for 20 hours in a culture medium containing the L-ascorbic acid derivative in a concentration of 5 µM, an L-ascorbic acid intracellular cumulative amount equal to or greater than that obtained by culturing the zooblast in a culture medium containing magnesium L-ascorbic acid-2-phosphate in a concentration of 50 µM.

12. A pharmaceutical preparation, for inhibiting cancer metastasis, which comprises at least one L-ascorbic acid derivative represented by the following formula (1) and pharmaceutically acceptable salts thereof: 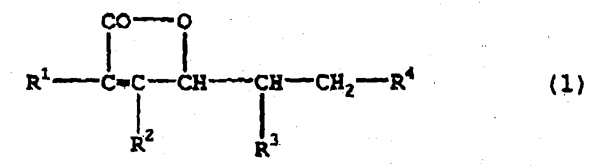 wherein R¹ is a substituent bonded to the 2-position capable of being biotransformed into a hydroxy group; R² represents a hydroxyl group or a substituent capable of being biotransformed into a hydroxyl group; and at least one of R³ and R⁴ is a hydrophobic group and the other is a hydroxyl group or a hydrophobic group.

13. The pharmaceutical preparation according to claim 12, wherein in formula (1), R¹ in one of a phosphoryloxy group, a pyrophosphoryloxy group, a triphosphoryloxy group, a polyphosphoryloxy group, a sulfoxy group and a glycosyloxy group, R² is one of a hydroxyl group, a phosphoryloxy group, a polyphosphoryloxy group, a sulfoxy group, a glycosyloxy group, an alkoxy group, an alkenyloxy group and a phenoxy group, and at least one of R³ and R⁴ is an acyloxy group, an alkoxy group, an alkenyloxy group or a phenoxy group.

14. The pharmaceutical preparation according to claim 12, wherein in formula (1), R¹ is a phosphoryloxy group, and R⁴ is an acyloxy group having from 10 to 22 carbon atoms

15. A method of treating cancer, which comprises administering to a person in need of such treatment a dosage effective amount of the pharmaceutical preparation according to claim 3.

16. The method according to claim 15, wherein in formula (1), R¹ is one of a phosphoryloxy group, a pyrophosphoryloxy group, a triphosphoryloxy group, a polyphosphoryloxy group, a sulfoxy group and a glycosyloxy group, R² is one of a hydroxyl group, a phosphoryloxy group, a polyphosphoryloxy group, a sulfoxy group, a glycosyloxy group, an alkoxy group, an alkenyloxy group and a phenoxy group, and et least one of R³ and R⁴ is an acyloxy group, an alkoxy group, an alkenyloxy group or a phenoxy group.
